# EUROPEAN PATENT APPLICATION

(11) **EP 3 808 432 A1**
(43) Date of publication of application: **21.04.2021**
(21) Application number: 19203588.9
(22) Date of filing: 16.10.2019
(51) Int. Cl.: B01D 53/14, B01D 53/18, B01D 53/58, C01C 1/12, C07C 273/16, C05C 3/00, C05C 9/00

(54) **APPARATUS AND METHOD FOR PROCESSING EXHAUST GAS**

(71) Applicant: Yara International ASA, 0277 Oslo (NO)
(72) Inventor: Cuelenaere, Paul J.M., 4634 VR Woensdrecht (NL); van der Have, Kees, 4571 HP Axel (NL)
(74) Representative: De Clercq & Partners

(57) **Abstract**

The present invention discloses an apparatus or scrubber to remove dust, in particular urea dust, and ammonia from a stream of polluted gas. The scrubber has an elongated shape along a central axis defined by two ends joined by a casing, and comprises one gas inlet, adapted to receive the stream of polluted gas, located on the casing of the scrubber, at least one gas outlet located on either end, adapted to exhaust a stream of gas depleted of dust and ammonia, at least two dust filter packings and at least two ammonia filter packings enclosed by the casing and adapted to pass the stream of polluted gas, wherein at least one dust filter packing is located on either side of the gas inlet, and at least one ammonia filter packing is located on either side of the gas inlet.

## Description

### Field of the invention

The present invention relates to the field of preparing urea fertilizers, and more specifically, to the field of processing exhaust gases from a urea plant, the exhaust gas comprising dust and ammonia. A scrubber to remove dust and ammonia from a stream of polluted gas is provided.

### Background of the invention

In chemical plants, scrubbers are used to remove pollutants from a stream of polluted gas. These pollutants can be divided in two groups according to their physical state: solid particles, which can have a diameter as small as a few nanometers up to hundreds of micrometers. These particles can be made of inorganic or organic compounds or salts and are collectively referred to as dust. The other group of pollutants is gases. If these gases are toxic or dangerous for the environment, strict emission levels are imposed by regulations in most countries. Examples of such gas pollutants are carbon dioxide, carbon monoxide, nitrogen oxides (NOx), sulfur oxides (SOx) and ammonia. If a production process creates a stream of gas containing these pollutants in amounts superior to the legal limits, the stream of gas has to be purified from these pollutants before being released in the atmosphere. This removal may be achieved by passing the stream of gas through a series of filters that are designed to capture the pollutants. A device housing such filters and designed to reduce the amount of pollutants in a stream of gas is called a scrubber.

Urea is one of the most important chemicals industrially produced today, around 200 million tons of urea are produced worldwide every year. Most of it (above 90% of total production) is used as a fertilizer in agriculture as a nitrogen source. Urea is produced by reacting ammonia and carbon dioxide. A concentrated melt of urea is produced and processed, for example by granulation or prilling, to provide urea in solid form. The solidification stage takes place in a granulation or prilling equipment where gas is used to cool the hot droplets of urea. During the process, the cooling gas becomes polluted with contaminants such as urea dust and ammonia and needs to be purified before being released in the atmosphere because of environmental issues and regulations. Scrubbers are a well-known class of engineering devices that may be used to clean a stream of polluted gas coming out of a urea production unit. A stream of polluted gas enters the scrubber and goes through a series of pads, filters or packings, which removes the pollutants. These filters may be wetted with an acidic, neutral or basic aqueous solution to increase the efficiency of the apparatus. A filter wetted with water can be used to remove urea dust and a filter with an acidic solution, sulfuric acid for example, will be more efficient at removing ammonia from the stream of gas. Ammonia reacts with the acid and forms a highly water-soluble salt, ammonium sulfate for example.

A standard urea scrubber comprises a main longitudinal direction where the polluted gas comes in at one end of the longitudinal direction and exits at the other end. It comprises at one least one urea dust filter and at least one ammonia filter. The size of the scrubber and the number and size of the filters depends on the production rate of the plant where it is installed, the amount of gas flow that needs to be purified and the amount of pollutants that needs to be removed from the stream of gas. This standard design is well adapted to the construction of new plants where the desired scrubber size is accommodated in the overall design of the plants.

However, it might not be suitable when an existing plant has to be modified, for example to increase its capacity or if the area available for the plant is limited. A sufficient amount of space might not be available, so there is a need to develop a new design of urea scrubbers.

### Summary of the invention

In one aspect, a scrubber for removing dust and ammonia from a stream of polluted gas, having an elongated shape along a central axis defined by two ends joined by a casing, comprising one gas inlet, adapted to receive the stream of polluted gas, located on the casing of the scrubber, at least one gas outlet located on either end, adapted to exhaust a stream of gas depleted of dust and ammonia, at least two dust filter packings and at least two ammonia filter packings enclosed by the casing and adapted to pass the stream of polluted gas, wherein at least one dust filter packing is located on either side of the gas inlet, and at least one ammonia filter packing is located on either side of the gas inlet, is provided.

In another aspect, a method to remove dust and ammonia from a stream of polluted gas, comprising the steps of: providing a scrubber according to the present disclosure; directing the stream of polluted gas into the gas inlet of the scrubber provided above; collecting the gas depleted of dust and ammonia from the at least two gas outlets of the scrubber for further processing.

In another aspect, a method to revamp an existing urea-producing unit comprising an existing scrubber configured to remove dust and ammonia from a stream of polluted gas, comprising the step of replacing the existing scrubber with a scrubber according to the present disclosure, is provided.

### Brief description of the figures

Figure 1 describes an embodiment of a scrubber according to the present disclosure.
Figure 2 describes an embodiment of a scrubber according to the present disclosure.
Figure 3 is a cross section of the scrubber described in Figure 2.

### Detailed description of the invention

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

All references cited in this description are hereby deemed to be incorporated in their entirety by way of reference.

As used herein, the following terms have the following meanings:
"A", "an", and "the" as used herein refers to both singular and plural referents unless the context clearly dictates otherwise. By way of example, "a compartment" refers to one or more than one compartment.
"About" as used herein referring to a measurable value such as a parameter, an amount, a temporal duration, and the like, is meant to encompass variations of +/-20 % or less, preferably +/-10 % or less, more preferably +/-5 % or less, even more preferably +/-1 % or less, and still more preferably +/-0.1 % or less of and from the specified value, in so far such variations are appropriate to perform in the disclosed invention. However, it is to be understood that the value to which the modifier "about" refers is itself also specifically disclosed.
"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

In a first aspect, a scrubber for removing dust and ammonia from a stream of polluted gas is provided. The scrubber has an elongated shape along a central axis defined by two ends joined by a casing, the scrubber comprises one gas inlet, adapted to receive the stream of polluted gas, located on the casing of the scrubber, at least one gas outlet located on either end, adapted to exhaust a stream of gas depleted of dust and ammonia, at least two dust filter packings and at least two ammonia filter packings enclosed by the casing and adapted to pass the stream of polluted gas, wherein at least one dust filter packing is located on either side of the gas inlet, and at least one ammonia filter packing is located on either side of the gas inlet.

A novel scrubber was designed wherein the scrubber comprises one gas inlet located on the casing of the scrubber and one gas outlet on each end of the scrubber. According to the prior art, a scrubber comprises one gas inlet which can be located on the casing or on one end of the scrubber and only one gas outlet located on the opposite end compared to the gas inlet.

The scrubber further comprises at least two dust filter packings and at least two ammonia filter packings. The at least two dust filter packings are located on either side of the gas inlet, and so are the at least two ammonia filter packings. This arrangement creates two scrubbing areas, each comprising at least one dust filter packings and at least one ammonia filter packings, within a single scrubber. When a stream of polluted gas is introduced into the scrubber through the gas inlet, the stream of gas will be divided into two sub-streams. Each sub-stream will go through at least a dust filter packing and at least an ammonia filter packing.

The polluted gas may comprise different types of gases, such as nitrogen, oxygen, carbon dioxide, carbon monoxide, noble gases, such as helium and argon. It comprises dust and ammonia as pollutants, but may also comprise other types of pollutants, for examples, sulfur oxides (SOₓ), or nitrogen oxides (NOₓ). Dust refers to any kind of small solid particles, for example urea particles. In one embodiment, the stream of polluted gas comes from a urea-producing unit, for example a granulation equipment, such as a fluidized bed granulator or a prilling tower, or a cooling equipment.

In one embodiment, the polluted gas may be atmospheric air and may comprise nitrogen (about 78 volume%), oxygen (about 21 volume%), argon (about 1 volume%), carbon dioxide, dust and ammonia.

The dust filter packings may be wetted with water. It is well known in the art to use an aqueous solution to help capture the dust particles. The solution may be basic, neutral or acidic.

The ammonia filter packings may be wetted with an acidic aqueous solution. Typically, an aqueous diluted solution of phosphoric acid, nitric acid or sulfuric acid may be used. Other organic or inorganic acids are also suitable to be used in the ammonia filter packings.

The performance of a scrubber depends largely of the size of the scrubber and the amount of pollutants that needs to be removed from the stream of polluted gas. By dividing the stream of polluted gas into two sub-streams, it is possible to treat twice the amount of gas or pollutants as a prior art scrubber with the same diameter. Said otherwise, it is possible to treat the same amount of gas as with a prior art scrubber with a the scrubber according to the invention having a diameter smaller (for example from about 20% to about 50% smaller, in particular from about 25% to about 40% smaller) than the prior art design.

The new design requires that two gas pipes must be installed outside of the scrubber to recover the stream of gas exiting at the at least gas outlet on either end of the scrubber. The two gas pipes may be subsequently joined together to facilitate the release of the gas stripped of pollutants. These two pipes might complicate the installation of such a scrubber, but this minor inconvenient is largely outweighed by the benefits of the scrubber explained above.

In one embodiment, the gas inlet is located essentially in the middle of the central axis of the scrubber. It may be an advantage that the scrubber is divided in two essentially equal parts for stability issues and ease of operation.

In one embodiment, two gas outlets are located on either ends of the scrubber. It may be favorable to have two gas outlets on either side of the scrubber rather than one gas outlet. By having more outlets on each end, it allows the outlets and the pipes connected to them to be smaller. This may facilitate the incorporation of the scrubber in plants with different sizes of pipes.

In one embodiment, the scrubber comprises at least four dust filter packings, wherein at least two dust filter packings are located on either side of the gas inlet. If the stream of polluted gas contains a high concentration of dust particles, one dust filter packing might not be enough to achieve the desired concentration, so that at the two sub-streams of gas should pass through at least two dust filter packings each. In one embodiment, the scrubber comprises at least six dust filter packings, wherein at least three dust filter packings are located on either side of the gas inlet.

In one embodiment, the scrubber comprises at least four ammonia dust filter packings, wherein at least two ammonia filter packings are located on either side of the gas inlet. If the stream of polluted gas contains a high concentration of ammonia, one ammonia filter packing might not be enough to achieve the desired concentration. The two sub-streams of gas should pass through at least two ammonia filter packings each. In one embodiment, the scrubber comprises at least six ammonia filter packings, wherein at least three ammonia filter packings are located on either side of the gas inlet.

In one embodiment, the scrubber is configured so that the stream of polluted gas passes through the at least one dust filter packing first and subsequently the at least one ammonia filter packing. It is preferable to first remove dust particles, for example, urea dust particles, before removing ammonia. It ensures a better performance of the ammonia filter packings since the stream of gas is already cleared of some of the pollutants. Ammonia is a gas and is not absorbed by the dust filter packings.

In one embodiment, the positions of the dust filter packings on one side of the gas inlet are mirror images of the positions of the dust filter packings located on the other side of the gas inlet, relative to the position of the gas inlet. It may be an advantage that the two scrubbing sections of the scrubber are exact mirror images of each other for stability issues and ease of operation. If the two sections are identical, they will require maintenance at the same moment, thus stopping the operation only once per cycle. It is anticipated that the filter packings on both sides will show the same degree of wear and tear at any time. In one embodiment, the positions of the ammonia filter packings on one side of the gas inlet are mirror images of the positions of the ammonia filter packings located on the other side of the gas inlet.

In one embodiment, the scrubber is a rectangular cuboid, i.e. the cross-section of the body along the central axis is a rectangle. In one embodiment, the cross-section of the body along the central axis may be a square. The filter packings, for dust or ammonia, are commercially available in rectangular shape, so a rectangular cuboid shape for the body of the scrubber is preferred to easily fit the packings into a body. A rectangular cuboid is a polyhedron or six-sided prism where all angles are right angles and opposite faces are equal.

In another aspect, a method to remove dust and ammonia from a stream of polluted gas, comprising the steps of: providing a scrubber according to the present disclosure; directing the stream of polluted gas into the gas inlet of the scrubber provided above; collecting the gas depleted of dust and ammonia from the at least two gas outlets of the scrubber for further processing.

Once collected from the outlet of the scrubber, the stream of gas depleted of dust and ammonia may be released in the atmosphere or go through another purification process to remove other types of pollutants. The stream of gas may also be re-used in another process.

In another aspect, a method to revamp an existing urea-producing unit comprising an existing scrubber configured to remove dust and ammonia from a stream of polluted gas, comprising the step of replacing the existing scrubber with a scrubber according to the present disclosure, is provided.

It may be desirable to increase the amount of polluted gas that a urea plant can process. Building a new urea plant is very expensive and can cost up to hundreds of millions of euros or dollars. In order to produce more urea, it may be desirable to improve the capacity of an existing plant rather than building a new one. Increasing the capacity of the synthesis part of the plant means that a larger stream of polluted gas will be produced along the increased urea production. It may also happen that new local regulations are introduced that require a plant to further reduce the levels of dust or ammonia in the gas rejected in the atmosphere by a plant. With a scrubber according to the present disclosure, it is possible to increase the gas treatment capacity of the plant without increasing the footprint of the scrubber, i.e. the amount of floor space the scrubber is occupying. Indeed a scrubber according to the present disclosure with the same diameter of a standard scrubber design will be able to process up to twice the amount of polluted gas because of the presence of two scrubbing sections within a single scrubber. So it would be very advantageous and much more economical to replace a scrubber with a standard design with a scrubber according to the present disclosure.

In one embodiment, the scrubber according to the present disclosure has a diameter that is from about 20% to about 50% smaller, in particular from about 25% to about 45% smaller, than the diameter of the scrubber to be replaced. In some situations, it may not be necessary to increase the processing capacity of the scrubber but simply to replace an existing scrubber with a new scrubber. Using a scrubber according to the present disclosure will allow replacing an existing scrubber with a scrubber having a diameter up to half of the diameter of the existing scrubber. This gain of space can be used to perform other improvements to the plant and may be highly beneficial to the plant operations. It can free up space to introduce new elements in the plant, or improve the working conditions, or improve the safety of the plant operations.

The embodiments of the compositions and methods according to the invention will now be illustrated by means of the following non-limitative examples.

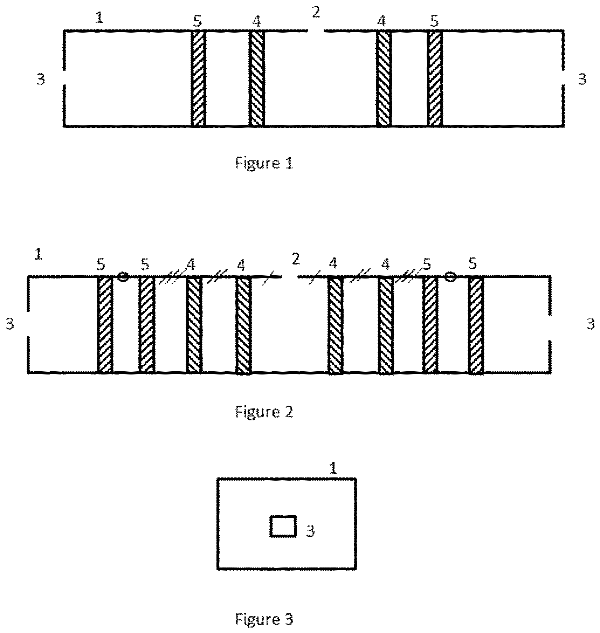

Figure 1 represents an embodiment of the present invention. The elongated scrubber body **1** comprises three inlets or outlets. One gas inlet **2** is located on the side of the body **1** located approximately in the middle of the central axis of the body. A stream of gas polluted with dust and ammonia can be fed to the scrubber via this inlet. Each end of the body comprises one gas outlet **3** where the stream of gas depleted of dust and ammonia can be collected to be disposed in the atmosphere or further processed. Inside the body **1** are located two dust filter packings **4** and two ammonia filter packings **5.** The two dust filter packings, respectively ammonia filter packings, are located on either side of the gas inlet **3.** The packings are disposed in such a way that the stream of gas entering the scrubber via the gas inlet **1** goes through the dust filter packings before the ammonia filter packings. The positions of the two dust filter packings, respectively ammonia filter packings, are mirror of each other with regards to the gas inlet.

Figure 2 represents another embodiment of the present invention. The rectangular cuboid scrubber body **1** comprises three inlets or outlets. One gas inlet **2** is located on the side of the body **1** located approximately in the middle of the central axis of the body. A stream of gas polluted with dust and ammonia can be fed in the scrubber via this inlet. Each end of the body comprises one gas outlet **3** where the stream of gas depleted of dust and ammonia can be collected to be disposed in the atmosphere or further processed. Inside the body **1** are located four dust filter packings **4** and four ammonia filter packings **5.** The four dust filter packings **4,** respectively ammonia filter packings **5,** are located on either side of the gas inlet **3** and the positions of the dust filter packings, respectively ammonia filter packings, on one side of the gas inlet are mirror images of the positions of the dust filter packings, respectively ammonia filter packings, located on the other side of the gas inlet. The packings are disposed in such a way that the stream of polluted gas entering the scrubber via the gas inlet **1** goes through the dust filter packings before the ammonia filter packings.

## Claims

1. A scrubber for removing dust and ammonia from a stream of polluted gas, having an elongated shape along a central axis defined by two ends joined by a casing, comprising one gas inlet, adapted to receive the stream of polluted gas, located on the casing of the scrubber, at least one gas outlet located on either end, adapted to exhaust a stream of gas depleted of dust and ammonia, at least two dust filter packings and at least two ammonia filter packings enclosed by the casing and adapted to pass the stream of polluted gas, wherein at least one dust filter packing is located on either side of the gas inlet, and at least one ammonia filter packing is located on either side of the gas inlet.

2. Scrubber according to claim 1, wherein the gas inlet is located essentially in the middle of the central axis of the scrubber.

3. Scrubber according to claim 1 to 2, wherein two gas outlets are located on either ends of the scrubber.

4. Scrubber according to any one of claims 1 to 3, wherein the scrubber comprises at least four dust filter packings, wherein at least two dust filter packings are located on either side of the gas inlet.

5. Scrubber according to any one of claims 1 to 4, wherein the scrubber comprises at least four ammonia filter packings, wherein at least two packings are located on either side of the gas inlet.

6. Scrubber according to any one of claims 1 to 5, wherein the scrubber is configured so that the stream of polluted gas passes through the at least one dust filter packing first and subsequently the at least one ammonia filter packing.

7. Scrubber according to any one of claims 1 to 6, wherein the positions of the dust filter packings on one side of the gas inlet are mirror images of the positions of the dust filter packings located on the other side of the gas inlet.

8. Scrubber according to any one of claims 1 to 7, wherein the position of the ammonia filter packings on one side of the gas inlet are mirror images of the position of the ammonia filter packing located on the other side of the gas inlet.

9. Scrubber according to any one of claims 1 to 8, wherein the scrubber is a rectangular cuboid.

10. A method to remove dust and ammonia from a stream of polluted gas, comprising the steps of:
a) providing a scrubber as defined in any one of claims 1 to 9;
b) directing the stream of polluted gas into the gas inlet of the scrubber provided in step a);
c) collecting the gas depleted of dust and ammonia from the at least two gas outlets of the scrubber for further processing.

11. A method to revamp an existing urea-producing unit comprising an existing scrubber configured to remove dust and ammonia from a stream of polluted gas, comprising the step of replacing the existing scrubber with a scrubber according to any one of claims 1 to 9.

12. Method according to claim 10, wherein the scrubber according to any one of claims 1 to 9 has a diameter that is from about 20% to about 50% smaller, in particular from about 25% smaller to about 45% smaller, than the diameter of the existing scrubber.
